# EUROPEAN PATENT SPECIFICATION

(11) **EP 2 237 825 B1**
(45) Date of publication and mention of the grant of the patent: **03.08.2016**
(21) Application number: 09701414.6
(22) Date of filing: 12.01.2009
(51) Int. Cl.: A61M 15/00

(54) **IMPROVEMENTS IN OR RELATING TO INHALERS**
VERBESSERUNGEN AN ODER IM ZUSAMMENHANG MIT INHALATOREN
PERFECTIONNEMENTS À DES INHALATEURS OU SE RAPPORTANT À DES INHALATEURS

(30) Priority: 11.01.2008 GB 0800459
(43) Date of publication of application: 13.10.2010
(73) Proprietor: Innovata Biomed Limited, Edinburgh EH1 2EN (GB)
(72) Inventor: BRIANT, John Philip, Edinburgh EH1 2EN (GB); MCDERMENT, Iain, Grierson, Edinburgh EH1 2EN (GB); MARTYN, Glen Patrick, Edinburgh EH1 2EN (GB)
(74) Representative: Adamson Jones
(86) International application number: PCT/GB2009/000077
(87) International publication number: WO 2009/087407

(56) References cited:
- EP-A- 1 300 171
- WO-A1-90/13328
- US-A1- 2003 116 157
- US-A1- 2003 136 405
- US-A1- 2007 246 044
- US-B1- 6 810 873

## Description

This invention concerns improvements in or relating to inhalers, and in particular to improvements in dry powder inhalers, that is to say devices for the administration of powdered medicament by inhalation, and especially to improvements that reduce the susceptibility of such devices to moisture ingress.

The administration of medicaments by inhalation is well-known. A wide variety of medicaments are now administered by that route, for the treatment of a range of respiratory disorders.

Examples of medicaments used for the treatment of respiratory disorders include, among others, anti-allergic agents, eg cromoglycate, ketotifen and nedocromil; anti-inflammatory steroids, eg beclomethasone dipropionate, fluticasone, budesonide, flunisolide, ciclesonide, triamcinolone acetonide and mometasone furoate; bronchodilators such as ß₂-agonists, eg fenoterol, formoterol, pirbuterol, reproterol, salbutamol, salmeterol, indacaterol and terbutaline, non-selective ß-stimulants, eg isoprenaline, and xanthine bronchodilators, eg theophylline, aminophylline and choline theophyllinate; anti-muscarinics, eg trospium chloride and glycopyrrolate (racemate and enantiomers thereof); PDE4 inhibitors, eg cilomilast and roflumilast; calcium channel blockers, eg diltiazem, verapamil, amlodipine, felodipine, lercanidipine and nimodipine; and anticholinergic agents, eg ipratropium bromide, oxitropium bromide and tiotropium.

The most common form in which such medicaments are formulated for administration by inhalation is as a powder. In the past, many such compositions were formulated as pressurised aerosols, in which the powder medicament was suspended in a liquefied propellant. Due to the adverse environmental effects of the propellants conventionally used, however, there is now increased interest in the use of so-called dry powder inhalers (DPIs). In a DPI, a unit dose of medicament powder, either packaged as such or metered from a bulk reservoir of medicament, is presented to an airway and is then entrained in an airflow passing through the airway. The airflow is most commonly generated by the patient's act of inhalation.

For the effective treatment of conditions of the respiratory tract it is generally desirable that as high a proportion of the powder as possible should be in the form of particles that are sufficiently fine that they are able to penetrate deep into the airways, and in particular that they should be transported deep into the lung. An important parameter in assessing the effectiveness of powdered medicament intended for inhalation is therefore the fine particle fraction (FPF), which defines the fraction of the emitted dose from an inhaler that has the potential to be deposited in the lung. This fraction is often defined as the proportion of the medicament that is in the form of particles with a diameter of less than 5µm.

The FPF will depend to some extent on the manner in which the medicament is formulated, but also is strongly dependent on the performance of the device (inhaler) from which the formulation is delivered. It is well known that one problem that can affect the performance of a dry powder inhaler is the ingress of moisture into the device, and in particular into the parts of the inhaler in which medicament is stored prior to delivery to a user of the inhaler. Such problems are particularly acute when the medicament is hygroscopic and/or when the inhaler is stored or used in climatic conditions that give rise to high humidity. Ingress of moisture into dry powder inhalers is therefore generally undesirable as it may lead to agglomeration of the powdered medicament, resulting in reduction of FPF, and potentially to clogging of the device and/or inaccurate dosing of the medicament. Furthermore, some medicaments are susceptible to chemical degradation in the presence of moisture.

There is therefore an ongoing requirement to improve the resistance of inhaler devices to the ingress of moisture.

There have now been devised improvements to dry powder inhalers that result in reduced ingress of moisture into the inhaler, and which may hence improve the performance of the inhaler.

Thus, according to a first aspect of the invention there is provided a dry powder inhaler comprising at least one medicament reservoir containing a bulk quantity of a powdered medicament, and a metering mechanism by which unit doses of said medicament can be dispensed from the medicament reservoir for inhalation by a user of the inhaler, wherein said at least one medicament reservoir is formed in polyester material, and the polyester material comprises polyethylene terephthalate (PET) or a related material, or polycarbonate or polybutyrate.

The applicant has found that the use of polyester material in the manufacture of the inhaler according to the invention is beneficial in that penetration of moisture through the walls of the medicament reservoir is considerably reduced.

Thus, according to another aspect of the invention, there is provided a method of reducing the ingress of moisture into the medicament reservoir of a dry powder inhaler, which method comprises forming the medicament reservoir in polyester material. Similarly, the invention provides the use of polyester material in the manufacture of the medicament reservoir of a dry powder inhaler.

Reduction of the ingress of moisture into the medicament reservoir may lead to an improvement in the FPF of the medicament inhaled by a user of the device. Thus, the invention further provides a method of improving the fine particle fraction of medicament dispensed from a dry powder inhaler comprising a medicament reservoir containing a bulk quantity of a powdered medicament, which method comprises forming the medicament reservoir in polyester material.

Where the medicament reservoir comprises more than one component, the component(s) that account for the majority of the external surface area of the medicament reservoir are formed in polyester material. Other, minor components (eg closures) may or may not be formed in polyester material.

Polyesters that may be used in the manufacture of the medicament reservoir are preferably polyethylene terephthalate (PET) or related materials. PET is obtained by a polycondensation reaction of the monomer normally obtained either by esterification of terephthalic acid (benzene-1,4-dicarboxylic acid) with ethylene glycol, or by transesterification of dimethyl terephthalate with ethylene glycol.

A preferred material that can be used in the present invention is glycol-modified PET (PET-G), ie a form of PET in which some of the ethylene glycol residues in the polymer backbone are replaced by other residues. For instance, cyclohexane dimethanol residues may be included in place of ethylene glycol units.

Another modification that can be made is the replacement of some of the terephthalic acid (benzene-1,4-dicarboxylic acid) residues of the polymer backbone with isophthalic acid (benzene-1,3-dicarboxylic acid) and/or phthalic acid (benzene-1,2-dicarboxylic acid).

Apart from PET, other polyester materials that may be used in the present invention include polycarbonate and polybutyrate.

A particularly preferred material for use in the present invention is the glycol-modified PET sold under the trade name EASTAR MN200 by Eastman Chemical Company (PO Box 431, Kingsport, TN 37662, USA).

Another polyester material that may be suitable for use in the invention is that sold by Eastman Chemical Company under the trade name EASTAR MN005.

Dry powder inhalers in which the inventive measures described herein may be particularly appropriate include those that comprise a medicament reservoir and a metering member that closes an opening in the reservoir and which is adapted to transfer a unit dose of medicament from the reservoir to a position from which the dose of medicament can be inhaled by the user of the device. Examples of such devices are those referred to by the names DUOHALER^{®} and CLICKHALER^{®}. The principle of operation of such devices is described in WO-A-92/00771 and WO-A-01/39823. The device includes a medicament reservoir (or more than one such reservoir), the lower end of which is closed by a rotatable metering member that is formed with a series of cups that are sized such that they can hold a unit dose of the medicament. Rotation of the metering member moves a cup from a first position in which it fills with medicament to a second position, external to the reservoir, at which the cup is positioned adjacent to the path of an airflow through the device, by means of which the dose can be entrained in the airflow and inhaled. In the DUOHALER^{®} and CLICKHALER^{®}, the metering members take the form of frustoconical members that are urged into close engagement with the lower part of the respective reservoirs. In other devices, the metering members may take other forms, eg members that move in a linear or reciprocating manner.

Apart from the medicament reservoir(s), the components of the inhaler according to the invention may be manufactured using conventional materials. However, it may be advantageous for certain components, in particular those that, together with the medicament reservoir(s), isolate the medicament from the external environment, also to be formed in polyester material. Thus, in embodiments of the invention, the medicament reservoir is formed in polyester material, as is a metering member that cooperates with the medicament reservoir to close the same and to transfer a unit dose of medicament from the reservoir to a position from which the dose can be inhaled, and/or a closure that is engaged with the medicament reservoir after the reservoir is filled with medicament. Where components other than the medicament reservoir are formed in polyester material, the type or grade of polyester material used may be the same as that used for the medicament reservoir, or it may be different. For instance, the medicament reservoir may be formed in the glycol-modified PET sold under the trade name EASTAR MN200, and the metering member and/or the closure in the glycol-modified PET sold under the trade name EASTAR MN005 (both Eastman Chemical Company).

The components of dry powder inhalers according to the invention may be manufactured by conventional techniques. Most commonly, the components will be formed in plastics materials, by injection moulding processes.

The inhaler according to the invention may be used to deliver any of a wide range of medicaments. Such medicaments include those that are suitable for the treatment of asthma, COPD and respiratory infections. Examples of such medicaments are given on page 1 hereof. Other classes of medicaments with which the inhaler may be used include the following:
- antivirals, eg zanamivir, ribavirin, flumist, ruprintrivir and pleconaril; antibiotics, eg tobramycin, doripenem, pentamidine, promixin, aztreonam; and antifungals, eg amphotericin B (further examples of medicaments that may potentially be administered using inhalers according to the invention are given in Respiratory Care 2000; 45(7): 836-845);
- immunogens for the prevention or treatment of meningococcal disease, meningitis, septicaemia, meningoccaemia, pneumonia, meningococci of any of groups A, B, C, Y, W135, X and/or Z, anthrax, plague, small pox, tularaemia, meliodosis, Q fever, botulism, typhus, cholera, yellow fever, brucellosis, encephalitis, ricin, salmonella, staphylococcal Enterotoxin B, human immunodeficiency virus, hepatitis B, cytomegalovirus, tuberculosis, and combinations thereof;
- proteinaceous compounds, macromolecules, hormones, mediators, insulin, human growth hormone, leuprolide, alpha-interferon, growth factors, anticoagulants, immunomodulators, cytokines, nucleic acids and combinations thereof;
- antimigraine compounds, particularly triptans. Preferably, the triptan comprises almotriptan, rizatriptan, naratriptan, zolmitriptan, sumatritpan, eletriptan, frovatriptan, and combinations thereof.

Inhalers according to the invention may also be used to deliver combinations of two or more different medicaments. Specific combinations of medicaments which may be mentioned include combinations of steroids and ß₂-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; and mometasone and formoterol. Another preferred combination is indacaterol and glycopyrrolate. Triple combinations include glycopyrrolate, salmeterol and mometasone; glycopyrrolate, indacaterol and mometasone; glycopyrrolate, salmeterol and ciclesonide; glycopyrrolate, indacaterol and ciclesonide. Some or all of the constituents of such combinations can be co-formulated and stored in a single medicament reservoir, but more preferably the individual medicaments that make up the combination are stored in separate reservoirs within the inhaler.

The invention will now be described in greater detail, by way of illustration only, with reference to the accompanying drawings, in which
Figure 1 is a perspective view of a dry powder inhaler according to the invention;
Figure 2 is a perspective view of the internal components of the inhaler of Figure 1;
Figure 3 is a side view, partly in section and partly cut away, of a pair of medicament reservoirs and associated dispensing mechanisms that form part of the inhaler of Figure 1; and
Figure 4 is a graph illustrating the reduced level of moisture ingress into an inhaler according to the invention.

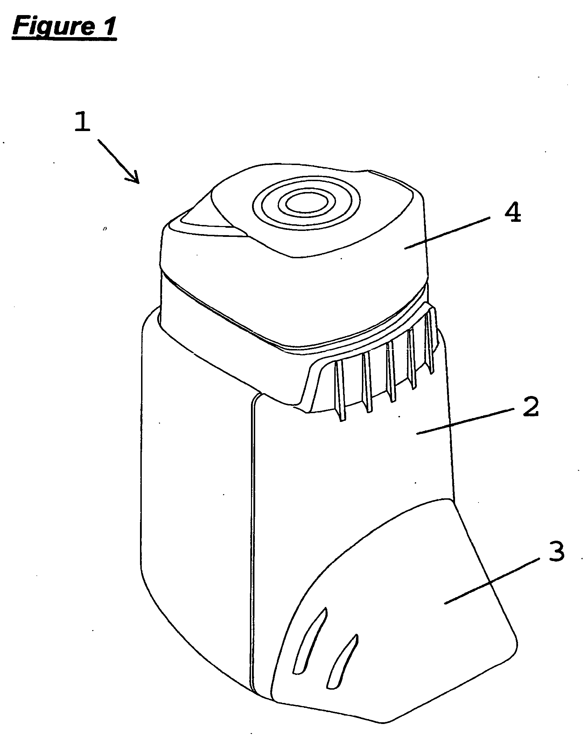

Referring first to Figure 1, a dry powder inhaler of known form is generally designated 1 and is of the type known by the name DUOHALER^{®}. The principle of action of such an inhaler is described in WO-A-01/39823 and in WO-A-2005/102429.

Essentially, the inhaler 1 comprises an inhaler body 2 with a mouthpiece that is covered by a removable shroud 3. The upper part of the inhaler 1 can be depressed relative to the inhaler body 2, the upper part thus constituting a push button 4 by means of which doses of medicament powders stored in bulk reservoirs within the inhaler body 2 can be dispensed from those reservoirs and inhaled by a user of the inhaler 1. Thus, in use, the user removes the shroud 3 to expose the mouthpiece, depresses the push button 4 to dispense unit doses of the medicaments, places the mouthpiece to his lips and inhales. The doses of medicament that have been dispensed from the bulk reservoirs are entrained in a flow of air through the inhaler 1 and are hence drawn into the user's lungs.

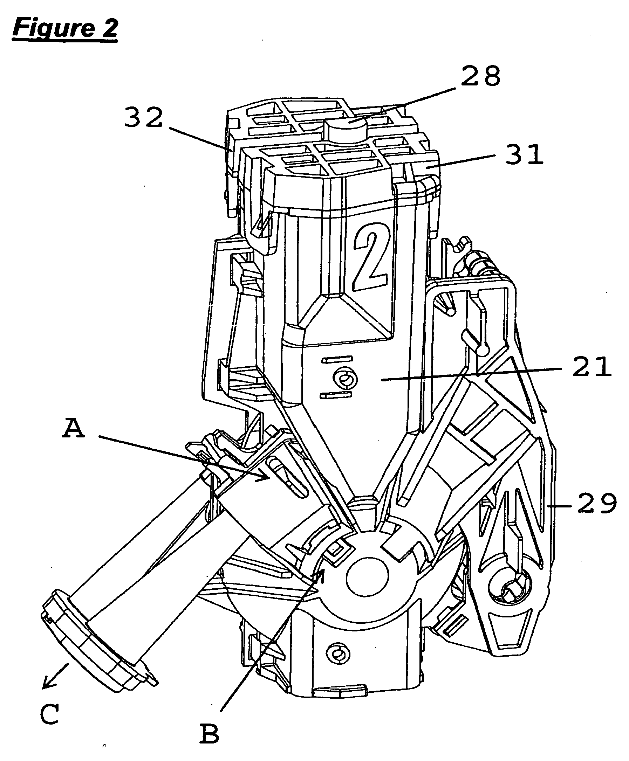

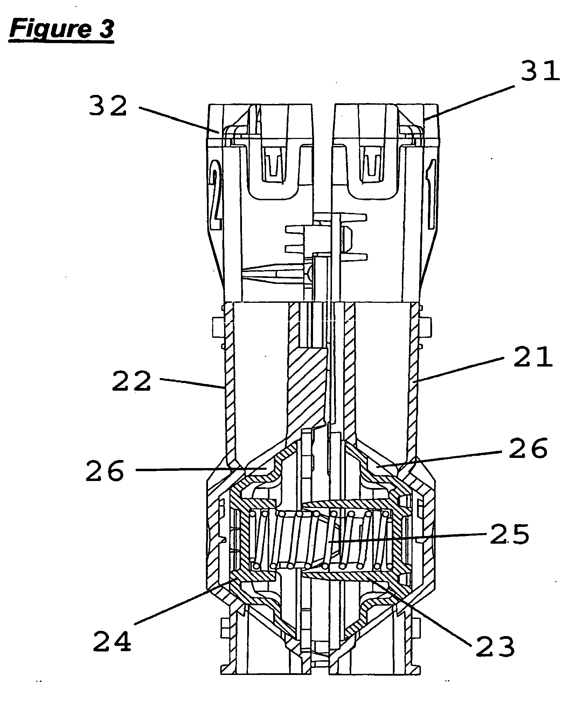

Figures 2 and 3 show the internal components of the inhaler 1 in greater detail. Specifically, the inhaler 1 comprises a pair of medicament reservoirs 21,22 that are arranged side-by-side. The lower end of each reservoir 21,22 is closed by a respective frustoconical metering member 23,24. The metering members 23,24 are mounted for rotation about a common axis that is transverse to the longitudinal axis of the reservoirs 21,22. Each metering member 23,24 is urged outwardly, into sealing engagement with its associated reservoir 21,22 by a compression spring 25.

Each metering member is formed with a series of cup-like depressions 26 in its surface, which depressions 26 serve for the volumetric metering of doses of medicament from each reservoir 21,22. In essence, the metering members 23,24 can be rotated in such a way that a depression 26 in each is brought into a filling position in which it is in registration with the bottom end of the associated reservoir 21,22, so that the depression 26 fills with a quantity of the medicament powder contained within that reservoir 21,22. Continued rotation then brings the filled depression into a dispensing position from which the dose of medicament can be entrained in an airflow (indicated by the arrows A, B and C in Figure 2) passing over the depression 26, the airflow being caused by the user inhaling through the mouthpiece. The airflow, in which the medicaments are entrained, passes through an airway 27 that is housed within the mouthpiece. When the depression 26 is moved from the filling to the dispensing position, the next depression 26 is brought into registration with the bottom end of the reservoir 21,22, ie into the filling position.

The abutting walls of the reservoirs 21,22 are formed with hemi-cylindrical channels that together form a cylindrical channel within which an actuator 28 is slidably mounted. The upper ends of reservoirs 21,22 are closed by respective caps 31,32.

The actuator 28 is acted upon by the push button 4 and serves to index the metering members 23,24 such that a depression 26 in each is moved from the filling to the dispensing position. A counter unit 29 is attached to the rear of the reservoirs 21,22 and provides a visual display of the number of actuations of the inhaler 1 at a window (not visible in Figure 1) in the rear of the inhaler body 2.

The manner in which the actuator 28 causes the metering members 23,24 to index and the manner in which the counter unit 29 records the number of actuations, as well as the details of the airway 27, are not pertinent to the present invention and will not be described in any greater detail.

The inhaler 1, as thus far described, is of known form. Specifically, the inhaler 1 is of the form known as DUOHALER^{®}, that has been developed by the present applicant, and which is a development of the inhaler sold under the name CLICKHALER^{®}, a multi-unit-dose reservoir device marketed for the delivery of several different medicaments in Europe and Japan. Such an inhaler allows the independent containment and delivery of two dry powder formulations simultaneously.

Whilst the performance of the known inhaler is good, the applicant has sought to develop the device further, and in particular has developed refinements to the device that improve the performance of the device by reducing the ingress of moisture into the reservoirs 21,22. This improved performance, in particular resistance to moisture ingress, may be of importance for the delivery of active ingredients that are especially sensitive to moisture, and may thus extend the utility of the inhaler 1 to the delivery of such medicaments.

In the inhaler of the present invention, the reservoirs 21,22 are formed of polyester,e g by injection-moulding in the glycol-modified polyester material EASTAR MN200. The metering members 23,24 and/or the caps 31,32 may also be formed in polyester, eg in the glycol-modified polyester material EASTAR MN005.

Tests of an inhaler according to the invention were performed, in comparison to the corresponding device in which the medicament reservoirs were formed in acrylonitrile-butadiene-styrene (ABS). The tests were performed by encapsulating the devices in resin blocks such that all possible routes of ingress of moisture, apart from direct ingress through the walls of the medicament reservoirs 21,22, were closed off. The encapsulated devices were stored at 100% RH and 21 °C and then weighed at intervals. Ingress of moisture was indicated by weight gain.

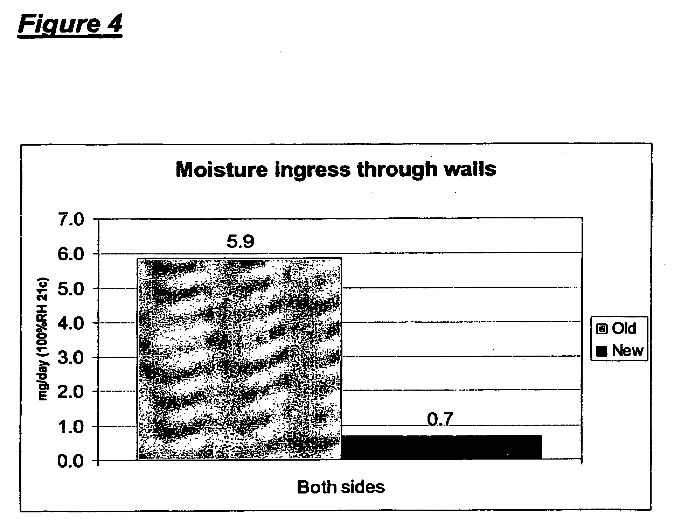

Figure 4 shows the weight gain (mg/day) for the inhaler according to the invention ("New") and the inhaler in which the reservoirs were formed in ABS ("Old"). It can be seen that the weight gain for the latter is 5.9mg/day, whereas for the inhaler in accordance with the invention the weight gain is only 0.7mg/day. Thus, it can be concluded that the use of glycol-modified PET as the material of the medicament reservoirs significantly reduces the level of moisture ingress during periods of storage under high humidity.

It will be appreciated that, in addition to the use of polyester material in accordance with the present invention, further measures may be taken to reduce the extent of moisture ingress into the inhaler. For instance, the measures described in our copending International patent application of even date (Attorney ref: 1620/1676/P/WO), claiming priority from United Kingdom patent application 0800457.4, may also be incorporated into the inhaler of the present invention.

## Claims

1. A dry powder inhaler comprising at least one medicament reservoir containing a bulk quantity of a powdered medicament, and a metering mechanism by which unit doses of said medicament can be dispensed from the medicament reservoir for inhalation by a user of the inhaler, wherein said at least one medicament reservoir is formed in polyester material, and the polyester material comprises polyethylene terephthalate (PET) or a related material, or polycarbonate or polybutyrate.

2. An inhaler as claimed in Claim 1, wherein the polyester material consists of polyethylene terephthalate (PET).

3. An inhaler as claimed in any preceding claim, wherein the polyester material is glycol-modified PET.

4. An inhaler as claimed in Claim 3, wherein the glycol-modified PET is a form of PET that contains cyclohexane dimethanol residues in place of ethylene glycol units.

5. An inhaler as claimed in any preceding claim, wherein the polyester material contains isophthalic acid (benzene-1,3-dicarboxylic acid) and/or phthalic acid (benzene-1,2-dicarboxylic acid) residues in place of some of the terephthalic acid (benzene-1,4-dicarboxylic acid) residues.

6. An inhaler as claimed in any preceding claim, which comprises a medicament reservoir and a rotatable metering member that closes an opening in the reservoir and is formed with a series of cups that are sized such that they can hold a unit dose of the medicament, such that rotation of the metering member moves a cup from a first position in which it fills with medicament to a second position, external to the reservoir, at which the cup is positioned adjacent to the path of an airflow through the device, by means of which the dose can be entrained in the airflow and inhaled.

7. An inhaler as claimed in Claim 6, wherein the rotatable metering member takes form of a frustoconical member that is urged into close engagement with the lower part of the medicament reservoir.

8. An inhaler as claimed in Claim 6 or Claim 7, wherein the metering member is formed in polyester material.

9. An inhaler as claimed in Claim 8, wherein the medicament reservoir is formed in the glycol-modified PET, and a metering member in the glycol-modified PET.

10. A method of reducing the ingress of moisture into the medicament reservoir of a dry powder inhaler of Claim 1, which method comprises forming the medicament reservoir in polyester material.

11. The use of polyester material in the manufacture of the medicament reservoir of a dry powder inhaler of Claim 1.

12. A method as claimed in Claim 10 or a use as claimed in Claim 11, wherein the inhaler is as claimed in any one of Claims 2 to 9.

## Patentansprüche

1. Trockenpulverinhalator, umfassend mindestens einen Arzneimittelbehälter mit einem Packungsinhalt eines pulverisierten Arzneimittels und einen Dosiermechanismus, mithilfe dessen Einheitsdosen dieses Arzneimittels aus dem Arzneimittelbehälter zur Inhalation durch einen Benutzer des Inhalators abgegeben werden können, wobei dieser mindestens eine Arzneimittelbehälter aus Polyestermaterial gebildet ist und das Polyestermaterial Polyethylenterephthalat (PET) oder ein verwandtes Material oder Polycarbonat oder Polybutyrat umfasst.

2. Inhalator nach Anspruch 1, wobei das Polyestermaterial Polyethylenterephthalat (PET) umfasst.

3. Inhalator nach einem der vorhergehenden Ansprüche, wobei das Polyestermaterial mit Glykol modifiziertes PET ist.

4. Inhalator nach Anspruch 3, wobei das mit Glykol modifizierte PET eine Form des PET ist, die Reste von Cyclohexandimethanol anstelle der Ethylenglykoleinheiten enthält.

5. Inhalator nach einem der vorhergehenden Ansprüche, wobei das Polyestermaterial Reste von Isophthalsäure (Benzol-1,3-dicarbonsäure) und/oder Reste von Phthalsäure (Benzol-1,2-dicarbonsäure) anstelle eines Teils der Reste der Terephthalsäure (Benzol-1,4-dicarbonsäure) enthält.

6. Inhalator nach einem der vorhergehenden Ansprüche, der einen Arzneimittelbehälter und ein drehbares Dosierelement umfasst, das eine Öffnung im Behälter schließt und aus einer Reihe von Bechern gebildet ist, die so dimensioniert sind, dass sie eine Einheitsdosis des Arzneimittels fassen können, so dass die Drehung des Dosierelements einen Becher von einer ersten Stellung, in der er mit dem Arzneimittel befüllt wird, in eine zweite Stellung außerhalb des Behälters bewegt, an der der Becher neben dem Luftstromweg durch das Gerät positioniert wird, wodurch die Dosis von dem Luftstrom mitgeführt und inhaliert werden kann.

7. Inhalator nach Anspruch 6, wobei das drehbare Dosierelement die Form eines Kegelstumpfelements hat, das unter Aufbringen von Kraft eng mit dem unteren Teil des Arzneimittelbehälters in Eingriff gebracht wird.

8. Inhalator nach Anspruch 6 oder Anspruch 7, wobei das Dosierelement aus Polyestermaterial gebildet ist.

9. Inhalator nach Anspruch 8, wobei der Arzneimittelbehälter aus mit Glykol modifiziertem PET und das Dosierelement aus mit Glykol modifiziertem PET gebildet sind.

10. Verfahren zur Reduzierung von Feuchtigkeitseintritt in den Arzneimittelbehälter eines Trockenpulverinhalators nach Anspruch 1, wobei das Verfahren die Bildung des Arzneimittelbehälters aus Polyestermaterial umfasst.

11. Verwendung von Polyestermaterial bei der Herstellung des Arzneimittelbehälters eines Trockenpulverinhalators nach Anspruch 1.

12. Verfahren nach Anspruch 10 oder Verwendung nach Anspruch 11, wobei der Inhalator nach jedem der Ansprüche 2 bis 9 ist.

## Revendications

1. Inhalateur de poudre sèche comprenant au moins un réservoir à médicament contenant une quantité de médicament sous forme de poudre en vrac et un mécanisme de dosage permettant de distribuer des doses unitaires dudit médicament à partir du réservoir à médicament lorsqu'un utilisateur de l'inhalateur doit s'administrer une inhalation, ledit au moins un réservoir à médicament étant fabriquer en matériau de polyester et ledit matériau de polyester comprenant du polyéthylène téréphtalate (PET) ou un matériau apparenté, ou un polycarbonate ou un polybutyrate.

2. Inhalateur selon la revendication 1, ledit matériau de polyester étant constitué de polyéthylène téréphtalate (PET).

3. Inhalateur selon l'une quelconque des revendications précédentes, ledit matériau de polyester étant du PET modifié au glycol.

4. Inhalateur selon la revendication 3, ledit PET modifié au glycol étant une forme de PET qui contient des résidus de cyclohexane diméthanol à la place des motifs d'éthylène glycol.

5. Inhalateur selon l'une quelconque des revendications précédentes, ledit matériau de polyester contenant des résidus d'acide isophtalique (acide benzène-1,3-dicarboxylique) et/ou d'acide phtalique (acide benzène-1,2-dicarboxylique) à la place d'une partie des résidus d'acide téréphtalique (acide benzène-1,4-dicarboxylique).

6. Inhalateur selon l'une quelconque des revendications précédentes qui comprend un réservoir à médicament et un élément de dosage rotatif qui ferme une ouverture dans le réservoir et est constitué d'une série de coupelles qui sont dimensionnées de façon à pouvoir contenir une dose unitaire du médicament, de sorte que la rotation de l'élément de dosage déplace une coupelle d'une première position dans laquelle elle se remplit de médicament à une seconde position, externe au réservoir, dans laquelle la coupelle est placée adjacente à la trajectoire d'un flux d'air à travers le dispositif, au moyen de quoi la dose peut être entraînée dans le flux d'air et être inhalée.

7. Inhalateur selon la revendication 6, ledit élément de dosage rotatif prenant la forme d'un élément tronconique qui est amené en contact étroit avec la partie inférieure du réservoir à médicament.

8. Inhalateur selon la revendication 6 ou 7, ledit élément de dosage étant fabriqué en matériau de polyester.

9. Inhalateur selon la revendication 8, ledit réservoir à médicament étant fabriqué en PET modifié au glycol et un élément de dosage en PET modifié au glycol.

10. Procédé permettant de réduire la pénétration d'humidité dans le réservoir à médicament dudit inhalateur de poudre sèche selon la revendication 1, ledit procédé comprenant la fabrication du réservoir à médicament en matériau de polyester.

11. Utilisation d'un matériau de polyester dans la fabrication du réservoir à médicament dudit inhalateur de poudre sèche selon la revendication 1.

12. Procédé selon la revendication 10 ou utilisation selon la revendication 11, ledit inhalateur étant selon l'une quelconque des revendications 2 à 9.
